# EUROPÄISCHE PATENTSCHRIFT

(11) **EP 3 746 183 B1**
(45) Veröffentlichungstag und Bekanntmachung des Hinweises auf die Patenterteilung: **23.08.2023**
(21) Anmeldenummer: 19701608.2
(22) Anmeldetag: 22.01.2019
(51) Int. Cl.: A61Q 17/04, A61K 8/06, A61K 8/35

(54) **O/W-EMULSION MIT PHOTOCHEMISCH STABILEM 4-(TERT.BUTYL)-4´-METHOXYDIBENZOYLMETHAN IN ÖLTRÖPFCHEN KLEINER 8 MIKROMETER**
O/W EMULSION COMPRISING PHOTOCHEMICALLY STABLE 4-(TERT.-BUTYL)-4'-METHOXYDIBENZOYLMETHANE IN OIL DROPLETS SMALLER THAN 8 MICROMETERS
ÉMULSION HUILE DANS L'EAU CONTENANT DU 4-(TERT.BUTYL)-4'-MÉTHOXYDIBENZOYLMÉTHANE PHOTOCHIMIQUEMENT STABLE INCORPORÉ DANS DES GOUTTELETTES D'HUILE DONT LA TAILLE EST INFÉRIEURE À 8 MICROMÈTRES

(30) Priorität: 02.02.2018 DE 102018201594
(43) Veröffentlichungstag der Anmeldung: 09.12.2020
(73) Patentinhaber: Beiersdorf AG, 20253 Hamburg (DE)
(72) Erfinder: SCHADE, Tatjana, 25866 Mildstedt/Rosendahl (DE); VOLBRICH, Heike, 22303 Hamburg (DE)
(74) Vertreter: Beiersdorf AG
(86) Internationale Anmeldenummer: PCT/EP2019/051444
(87) Internationale Veröffentlichungsnummer: WO 2019/149565

(56) Entgegenhaltungen:
- EP-A1- 2 359 801
- EP-A1- 3 093 004
- EP-A2- 2 286 908
- WO-A1-02/43672
- WO-A2-03/075879
- US-A1- 2013 011 348
- Sol Kaplan: "CRODASOME UV-A/B Sun Protectant for Hair", , 1. Oktober 1996 (1996-10-01), Seiten 1-2, XP055576452, UL Prospector Gefunden im Internet: URL:https://www.ulprospector.com/documents /5724.pdf?bs=1832&b=5236&st=20&r=la&ind=pe rsonalcare [gefunden am 2019-04-02]

## Beschreibung

Die vorliegende Erfindung betrifft eine kosmetische Öl-in Wasser-Emulsion mit definierter Tröpfchengröße enthaltend 4-(tert.-Butyl)-4'-methoxydibenzoylmethan.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Sonnenschutzmittel enthalten üblicherweise sowohl UV-A als auch UV-B-Filter, damit die Zubereitung die menschliche Haut vor dem gesamten Spektrum der UV-Strahlung schützt. Als UV-A-Filter enthalten die meisten Zubereitungen die Verbindung 4-(tert.-Butyl)-4'-methoxydibenzoylmethan. Kosmetische Sonnenschutzmittel, welche die UV-A-Filtersubstanz 4-(tert.-Butyl)-4'-methoxydibenzoylmethan enthalten, haben jedoch das Problem, das diese Verbindung nicht besonders photostabil ist und sich unter Einwirkung von UV-Strahlung zersetzt. Sonnenschutzmittel, die 4-(tert.-Butyl)-4'-methoxydibenzoylmethan als UV-Filter enthalten, müssen zu dessen Photostabilisierung daher weitere Stabilisatoren zugefügt werden. Zur Photostabilisierung von 4-(tert.-Butyl)-4'-methoxydibenzoylmethan wird nach dem Stande der Technik bevorzugt Octocrylen verwendet.

Der Nachteil des Standes der Technik besteht nun in dem Umstand, dass der Einsatz von Octocrylen, trotz der Zulassung durch die Genehmigungsbehörden, nicht ganz unumstritten ist und bei Bewertungen bei einigen Verbrauchermagazinen (z.B. Öko-Test) zu "Abwertungen" in der Benotung des Produktes führen. Begründet wird diese Negativ-Bewertung damit, dass einige Wissenschaftler vermuten, dass dieser UV-Filter möglicherweise hormonell wirksam sein könnte. Auch wenn, trotz des jahrzehntelangen weltweiten Einsatzes dieses UV-Filters in Sonnenschutzmitteln keine negativen Wirkungen am Menschen bekannt geworden sind, besteht bei den Verbrauchern der Wunsch, Zubereitungen mit derartigen Inhaltsstoffen zu meiden.

Es war daher die Aufgabe der vorliegenden Erfindung, die Nachteile des Standes der Technik zu beseitigen und ein Sonnenschutzmittel zu entwickeln, bei dem der photochemische Abbau von 4-(tert.-Butyl)-4'-methoxydibenzoylmethan unterdrückt wird. Idealerweise sollte die Aufgabe gelöst werden ohne dass Octocrylen als Lösungsmittel und Stabilisator eingesetzt wird.

Überraschend gelöst wird die Aufgabe durch eine kosmetische O/W-Emulsion enthaltend 4-(tert.-Butyl)-4'-methoxydibenzoylmethan, wobei mindestens 90 % der Öltröpfchen in der Emulsion eine Größe (Durchmesser) von unter 8 µm aufweisen, dadurch gekennzeichnet dass der oder die O/W-Emulgatoren gewählt werden aus der Gruppe der Verbindungen Glycerylstearatcitrat, Glycerylstearat (selbstemulgierend), Stearinsäure, Stearatsalze, Polyglyceryl-3 methylglycose-distearat, Natriumcetearylsulfat, Kaliumcetylphosphat, Polyglyceryl-10 Stearate , Natriumstearoylglutamat und der Emulgator bei der Herstellung der Emulsion der Wasserphase zugesetzt wird.

Überraschend gelöst wird die Aufgabe durch ein Verfahren zur Herstellung 4-(tert.-Butyl)-4'-methoxydibenzoylmethan enthaltender kosmetischer O/W-Emulsionen, wobei das Verfahren die folgenden Schritte umfasst:
i) Vereinigung von Öl- und Wasserphase,
ii) Voremulgieren,
iii) Zugabe von temperaturempfindlichen Inhaltsstoffen wie Parfümstoffen
iv) Nachemulgieren,
wobei durch die Nachemulgierung Öltröpfchen mit einer Tröpfchengröße gebildet werden, bei denen mindestens 90% der Öltröpfchen eine Größe (Durchmesser) von unter 8 µm aufweisen, dadurch gekennzeichnet dass der oder die O/W-Emulgatoren gewählt werden aus der Gruppe der Verbindungen Glycerylstearatcitrat, Glycerylstearat (selbstemulgierend), Stearinsäure, Stearatsalze, Polyglyceryl-3 methylglycose-distearat, Natriumcetearylsulfat, Kaliumcetylphosphat, Polyglyceryl-10 Stearate , Natriumstearoylglutamat und der Emulgator bei der Herstellung der Emulsion der Wasserphase zugesetzt wird.

Überraschend gelöst wird die Aufgabe darüber hinaus durch ein Verfahren zur Photostabilisierung von 4-(tert.-Butyl)-4'-methoxydibenzoylmethan in kosmetischen O/W-Emulsionen, wobei das 4-(tert.-Butyl)-4'-methoxydibenzoylmethan in Öltröpfchen eingearbeitet wird, die zu mindestens 90 % eine Größe (Durchmesser) von unter 8 µm aufweisen, dadurch gekennzeichnet dass der oder die O/W-Emulgatoren gewählt werden aus der Gruppe der Verbindungen Glycerylstearatcitrat, Glycerylstearat (selbstemulgierend), Stearinsäure, Stearatsalze, Polyglyceryl-3 methylglycose-distearat, Natriumcetearylsulfat, Kaliumcetylphosphat, Polyglyceryl-10 Stearate , Natriumstearoylglutamat und der Emulgator bei der Herstellung der Emulsion der Wasserphase zugesetzt wird.

Überraschend gelöst wird die Aufgabe nicht zuletzt durch die Verwendung von Öltröpfchen mit einer Tröpfchengröße (Durchmesser) von unter 8 µm in kosmetischen O/W-Emulsionen zur Photostabilisierung von 4-(tert.-Butyl)-4'-methoxydibenzoylmethan, dadurch gekennzeichnet dass der oder die O/W-Emulgatoren gewählt werden aus der Gruppe der Verbindungen Glycerylstearatcitrat, Glycerylstearat (selbstemulgierend), Stearinsäure, Stearatsalze, Polyglyceryl-3 methylglycose-distearat, Natriumcetearylsulfat, Kaliumcetylphosphat, Polyglyceryl-10 Stearate , Natriumstearoylglutamat und der Emulgator bei der Herstellung der Emulsion der Wasserphase zugesetzt wird.

Überraschend wurde darüber hinaus gefunden, dass die erfindungsgemäßen Emulsionen einen höheren Lichtschutzfaktor (SPF) aufweisen, als Zubereitungen mit größeren Emulsionströpfchen.

Zwar kennt der Stand der Technik durchaus kosmetischen O/W-Emulsionen mit 4-(tert.-Butyl)-4'-methoxydibenzoylmethan, doch blieb bisher unbeachtet, dass die Tröpfchengröße der Emulsion einen Einfluss auf die Photostabilität von 4-(tert.-Butyl)-4'-methoxydiben-zoylmethan hat.

Darüber hinaus kennt der Stand der Technik die WO 03/075879 A2, EP 2 359 801 A1, US 2013/011348 A1, EP 2 286 908 A2, WO 02/43672 A1, EP 3 093004 A1 sowie die Internet-Veröffentlichung von Sol Kaplan: "Crodasome UV-A/B Sun Protectant for Hair" vom 1.Oktober 1996, Seiten 1-2, die unter "www.ulprospector.com" zu finden ist, doch konnten diese Schriften ebenfalls nicht den Weg zur vorliegenden Erfindung weisen.

Im Rahmen der vorliegenden Offenbarung beziehen sich die Formulierungen "erfindungsgemäß", "erfindungsgemäße Zubereitung" etc. immer auf die erfindungsgemäßen Zubereitungen, Verfahren und Verwendungen, d.h. auch auf Zubereitungen, in denen die erfindungsgemäßen Verwendungen verwirklicht werden sowie Zubereitungen, mit denen die erfindungsgemäße Verfahren verwirklicht werden.

Die Tröpfchengröße der Öltröpfchen in der Emulsion wird dabei erfindungsgemäß mit Hilfe der Laserbeugungsmessung bestimmt.

Hierzu kann das Messsystem Mastersizer 2000 von der Firma Malvern verwendet werden. Die Proben werden hier in Wasser dispergiert und bei Raumtemperatur (20°) vermessen. Die Verteilung der Tröpfchengröße wird nach dem Modell der Mie-Theorie berechnet. Brechungsindex der Teilchen: 1,469 Absorption 0.

Brechungsindex Dispergiermittel Wasser: 1,330.

Vorteilhafte Ausführungsformen der vorliegenden Erfindung sind dadurch gekennzeichnet, dass die Emulsion mit einem Rotor-/Stator-Gerät oder einem Hochdruck-Homogenisator homogenisiert wird. Bevorzugt werden diese Geräte zur Nachemulgierung eingesetzt.

Vorteilhaft im Sinne der vorliegenden Erfindung ist es ferner, wenn die Zubereitung 4-(tert.-Butyl)-4'-methoxydibenzoylmethan in einer Konzentration von 1,0 bis 5,0 Gewichts-%, bezogen auf das Gesamtgewicht der Emulsion enthält.

Die erfindungsgemäß vorteilhaften Ausführungsformen der vorliegenden Erfindung sind dabei dadurch gekennzeichnet, dass die Zubereitung frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen) und 4-Methoxyzimtsäure-2-ethylhexylester (INCI Octylmethoxycinnamate).

Dabei sind die erfindungsgemäß vorteilhaften Ausführungsformen dadurch gekennzeichnet, dass der oder die Emulgatoren in einer Gesamtkonzentration von 0,2 bis 5 Gewichts-%, bezogen auf das Gesamtgewicht der Zubereitung eingesetzt werden.

Dabei ist der Einsatz des Emulgators Natriumstearoylglutamat erfindungsgemäß bevorzugt. Für diesen Emulgator beträgt die erfindungsgemäß bevorzugte Einsatzkonzentration von 0,2 bis 0,5 Gewichts-%, bezogen auf das Gesamtgewicht der Zubereitung

Erfindungsgemäß vorteilhaft ist es ferner, wenn die erfindungsgemäße Zubereitung frei ist von Parabenen, Methylisothiazolinon, Chlormethylisothiazolinon und DMDM-Hydantoin.

Erfindungsgemäß vorteilhaft ist es auch, wenn die erfindungsgemäße Emulsion dadurch gekennzeichnet ist, dass die Zubereitung einen oder mehrere UV-Filter enthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)ben-zolsulfonsäuresalze; 2-Methyl-5-(2-oxo-3-bornylidenmethyl)sulfonsäuresalze; 2,2'-Methylenbis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; Homomenthylsalicylat; 2-Ethylhexyl-2-hydroxybenzoat; Dimethicodiethylbenzalmalonat; 3-(4-(2,2-bis Ethoxycarbonylvinyl)-phe-noxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4 methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Dabei ist der Einsatz von 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); Ethylhexylsalicylat sowie 2-Phenylbenzimidazol-5-sulfonsäure und/oder deren Salzen, erfindungsgemäß bevorzugt.

Erfindungsgemäß besonders bevorzugt ist der Einsatz von 2,4-Bis-{[4-(2-ethyl-hexyloxy)-2-hy-droxy]-phenyl}-6-(4-methoxyphenyl)-1,3,5-triazin (INCI: Bis-Ethylhexyloxyphenol Methoxyphenyl Triazin). Diese Verbindung wird erfindungsgemäß vorteilhaft in einer Menge von 2 bis 5 Gewichts-%, bezogen auf das Gesamtgewicht der Zubereitung, eingesetzt.

Die erfindungsgemäß vorteilhaften Einsatzkonzentrationen betragen für
4,4',4"-(1,3,5-Triazin-2,4,6-triyltriimino)-tris-benzoësäure-tris(2-ethylhexylester) (INCI: Ethylhexyl Triazone) 2 bis 5 Gewichts-%,
2-Ethylhexyl 2-hydroxybenzoat (INCI: Ethylhexyl Salicylate) 3 bis 5 Gewichts-%,
2-Phenylbenzimidazol-5-sulfonsäure und/oder deren Salzen1 bis 4 Gewichts-%, jeweils bezogen auf das Gesamtgewicht der Zubereitung,

Es ist erfindungsgemäß von Vorteil, wenn die Zubereitung Ethanol, Phenoxyethanol, 4-Hydroxyacetophenon, 2-Methylpropan-1,3-diol, 1,2-Pentandiol, 1,2-Hexandiol, 1,2-Octandiol und/oder 1,2-Decandiol und/oder Ethylhexylglycerin enthält.

Erfindungsgemäß bevorzugt ist der Einsatz von 4-Hydroxyacetophenon, welcher in einer Konzentration von 0,01 bis 2 Gewichts-%, bezogen auf das Gesamtgewicht der Zubereitung, bevorzugt eingesetzt wird.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum und/oder Polyethylen, Nylon, Silica Diemthyl Silyate.

Es ist erfindungsgemäß bevorzugt, wenn die erfindungsgemäße Zubereitung Silica Dimethyl Silylate enthält.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindungsgemäß bevorzugt, wenn die Zubereitung Dibutyladipat, Dicaprylylcarbonat und/oder C12-C15 Alkylbenzoat enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Es ist dabei erfindungsgemäß bevorzugt, wenn die Zubereitung Xanthangummi, quervernetztes Acrylat/C10-C30 Alkylacrylat Polymer und/oder Vinylpyrrolidon/Hexadecen-Copolymer enthält.

Ein Gehalt an Glycerin von mindestens 5 Gewicht-%, bezogen auf das Gesamtgewicht der Zubereitung, ist erfindungsgemäß besonders vorteilhaft.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

Es ist ferner vorteilhaft im Sinne der vorliegenden Erfindung, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 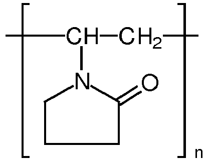 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

### Vergleichsversuche/Ausführungsbeispiele

Es wurden die folgenden Rezepturen hergestellt und die Photostabilität des 4-(tert.-Butyl)-4'-methoxydibenzoylmethan mit Hilfe der folgenden Methode bestimmt.

### Rezepturen:

| **INCI** | **sample A** | **Phase** | **sample B** | **Phase** | **sample C** | **Phase** | **sample D** | **Phase** |
|---|---|---|---|---|---|---|---|---|
| Isopropyl Palmitate | 7,00 | W | 7,00 | W | 7,00 | W | 7,00 | W |
| Aqua | 54,60 | O | 54,60 | O | 54,60 | O | 54,60 | O |
| Tocopheryl Acetate | 0,06 | O | 0,06 | O | 0,06 | O | 0,06 | O |
| Aqua+ Trisodium EDTA | 1,00 | W | 1,00 | W | 1,00 | W | 1,00 | W |
| Dimethicone | 0,90 | O | 0,90 | O | 0,90 | O | 0,90 | O |
| Panthenol + Aqua | 1,40 | W | 1,40 | W | 1,40 | W | 1,40 | W |
| Butyl Methoxydibenzoylmethane | 2,38 | O | 2,38 | O | 2,38 | O | 2,38 | O |
| Phenylbenzimidazole Sulfonic Acid | 0,50 | W | 0,50 | W | 0,50 | W | 0,50 | W |
| VP/Hexadecene Copolymer | 0,50 | O | 0,50 | O | 0,50 | O | 0,50 | O |
| Behenyl Alcohol | 1,00 | O | 1,00 | O | 1,00 | O | 1,00 | O |
| Glycerin + Aqua | 7,50 | W | 7,50 | W | 7,50 | W | 7,50 | W |
| Aqua + Sodium Hvdroxide | 0,11 | W | 0,11 | W | 0,11 | W | 0,11 | W |
| Alcohol Denat. + Aqua | 5,00 | A | 5,00 | A | 5,00 | A | 5,00 | A |
| Ethylhexyl Salicylate | 2,00 | O | 2,00 | O | 2,00 | O | 2,00 | O |
| Xanthan Gum | 0,40 | T | 0,40 | T | 0,40 | T | 0,40 | T |
| Silica Dimethyl Silylate | 1,00 | O | 1,00 | O | 1,00 | O | 1,00 | O |
| Stearyl Alcohol | 1,00 | O | 1,00 | O | 1,00 | O | 1,00 | O |
| Acrylates/C10-30 Alkyl Acrylate Crosspolymer | 0,10 | T | 0,10 | T | 0,10 | T | 0,10 | T |
| Ethylhexylglycerin | 0,20 | O | 0,20 | O | 0,20 | O | 0,20 | O |
| Bis-Ethylhexyloxyphenol Methoxyphenyl Triazine | 1,70 | O | 1,70 | O | 1,70 | O | 1,70 | O |
| Butylene Glycol Dicaprylate/Dicaprate | 3,00 | O | 3,00 | O | 3,00 | O | 3,00 | O |
| Tetrasodium Iminodisuccinate | 0,75 | W | 0,75 | W | 0,75 | W | 0,75 | W |
| Diethylamino Hydroxybenzoyl Hexyl Benzoate | 0,50 | O | 0,50 | O | 0,50 | O | 0,50 | O |
| Sodium Stearoyl Glutamate | 0,30 | O | 0,30 | O | 0,30 | W | 0,30 | W |
| C12-15 Alkyl Benzoate | 6,00 | O | 6,00 | O | 6,00 | O | 6,00 | O |
| Hydroxyacetophenone | 0,20 | W | 0,20 | W | 0,20 | W | 0,20 | W |
| Cellulose Gum | 0,50 | T | 0,50 | T | 0,50 | T | 0,50 | T |
| Parfum | 0,40 | A | 0,40 | A | 0,40 | A | 0,40 | A |
| | 100,00 | | 100,00 | | 100,00 | | 100,00 | |

| | |
|---|---|
| W= | Waterphase |
| O= | Oilphase |
| A= | Alcoholphase |
| T= | Thickener |

### Herstellung:

Die Phasen W und O werden getrennt auf 80-85°C erhitzt. Nach Zugabe der Phase T zu der Phase O werden, die beiden Phasen O und W in einem Mischer vereint. Nach Vereinigung werden die Ansätze unter Vakuum bei 75°C homogenisiert und auf 30-40°C gekühlt.

Nach Zugabe der Phase A werden 10min gerührt. Bei 30°C wird erneut Energie unter Vakuum durch Homogenisieren eingetragen und danach auf Raumtemperatur gekühlt.

Energie wurde nach folgendem Schema in die Ansätze eingetragen:

| | **sample A** | **sample B** | **sample C** | **sample D** |
|---|---|---|---|---|
| **Manufacturing** | | | | |
| Mischertyp | Becomix RW 125 | Becomix RW 125 | Becomix RW 125 | Conti Anlage |
| Engerieeintrag 75°C | 3min 14 m/s | 3min 14 m/s | 3min 14 m/s | |
| Engerieeintrag 30°C | 5min 25 m/s | 15min 25 m/s | 15min 25 m/s | |
| Energieeintrag Mixer 1 80°C [m/s] [1/min] | | | | 20 |
| Energieeintrag Mixer 2 75°C [m/s] [1/min] | | | | 3200 |
| Energieeintrag Mixer 1 30°C [m/s] [1/min] | | | | 10 |
| Energieintrag BTO (Batch Turn Over) | 1 | 3 | 3 | 3 |

### Bestimmung der Photostabilität

Es wurden die folgenden Rezepturen hergestellt und die Photostabilität des 4-(tert.-Butyl)-4'-methoxydibenzoylmethan bestimmt.

### Eingesetzte Methode(n):

Aufarbeitung und Analytik angelehnt an die COLIPA Methode "In Vitro Determination of Photostability of Suncare Products" vom Oktober 1999, aber ohne Vorbestrahlung. Dosis ca. 250 kJ/m²

### Dreifachbestimmung

| | |
|---|---|
| Gerät: | Atlas Suntest CPS+ mit Standardfilter und Wasserkühlung |
| | Gerät-Nr. 134653 |
| Dosis 290 - 400 nm: | Ca.250 kJ/m² (58min, 11s bei 765 W/m² Lampenleistung) |
| Auftragsmenge: | 2 mg/cm² |

### Als Standardsubstanz wurde Eusoles 9020 der Fa. Merck verwendet (#5844J093)

### Bestimmung der Tröpfchengröße:

Laserbeugungsmessung (Messsystem Mastersizer 2000 der Firma Malvern)
Die Proben wurden in Wasser dispergiert und bei Raumtemperatur (20°) vermessen. Die Verteilung der Tröpfchengröße wurde nach dem Modell der Mie-Theorie berechnet. Brechungsindex der Teilchen: 1,469 Absorption 0.
Brechungsindex Dispergiermittel Wasser: 1,330.

### Ergebnisse:

| | **sample A** | **sample B** | **sample C** | **sample D** |
|---|---|---|---|---|
| | | | | |
| Droplet size 90%(µm) | 10,3 | 8,5 | 7,9 | 5,5 |
| | | | | |
| SPF | 34,7 | 30,5 | 43,3 | 48,5 |
| | | | | |
| Recovery BMDM after Irradiation (250kJ/m²) [%] | 71 | 72 | 75 | 76 |

## Patentansprüche

1. Kosmetische O/W-Emulsion enthaltend 4-(tert.-Butyl)-4'-methoxydibenzoylmethan, wobei mindestens 90 % der Öltröpfchen in der Emulsion eine Größe (Durchmesser) von unter 8 µm aufweisen, **dadurch gekennzeichnet dass** der oder die O/W-Emulgatoren gewählt werden aus der Gruppe der Verbindungen Glycerylstearatcitrat, Glycerylstearat (selbstemulgierend), Stearinsäure, Stearatsalze, Polyglyceryl-3 methylglycose-distearat, Natriumcetearylsulfat, Kaliumcetylphosphat, Polyglyceryl-10 Stearate , Natriumstearoylglutamat und der Emulgator bei der Herstellung der Emulsion der Wasserphase zugesetzt wird.

2. Verfahren zur Herstellung 4-(tert.-Butyl)-4'-methoxydibenzoylmethan enthaltender kosmetischer O/W-Emulsionen, wobei das Verfahren die folgenden Schritte umfasst:
i) Vereinigung von Öl- und Wasserphase,
ii) Voremulgieren,
iii) Zugabe von temperaturempfindlichen Inhaltsstoffen wie Parfümstoffen
iv) Nachemulgieren,
wobei durch die Nachemulgierung Öltröpfchen mit einer Tröpfchengröße gebildet werden, bei denen mindestens 90% der Öltröpfchen eine Größe (Durchmesser) von unter 8 µm aufweisen, **dadurch gekennzeichnet, dass** der oder die O/W-Emulgatoren gewählt werden aus der Gruppe der Verbindungen Glycerylstearatcitrat, Glycerylstearat (selbstemulgierend), Stearinsäure, Stearatsalze, Polyglyceryl-3 methylglycose-distearat, Natriumcetearylsulfat, Kaliumcetylphosphat, Polyglyceryl-10 Stearate , Natriumstearoylglutamat und der Emulgator bei der Herstellung der Emulsion der Wasserphase zugesetzt wird.

3. Verfahren zur Photostabilisierung von 4-(tert.-Butyl)-4'-methoxydibenzoylmethan in kosmetischen O/W-Emulsionen, wobei das 4-(tert.-Butyl)-4'-methoxydibenzoylmethan in Öltröpfchen eingearbeitet wird, die zu mindestens 90 % eine Größe (Durchmesser) von unter 8 µm aufweisen, **dadurch gekennzeichnet, dass** der oder die O/W-Emulgatoren gewählt werden aus der Gruppe der Verbindungen Glycerylstearatcitrat, Glycerylstearat (selbstemulgierend), Stearinsäure, Stearatsalze, Polyglyceryl-3 methylglycose-distearat, Natriumcetearylsulfat, Kaliumcetylphosphat, Polyglyceryl-10 Stearate , Natriumstearoylglutamat und der Emulgator bei der Herstellung der Emulsion der Wasserphase zugesetzt wird.

4. Verwendung von Öltröpfchen mit einer Tröpfchengröße (Durchmesser) von unter 8 µm in kosmetischen O/W-Emulsionen zur Photostabilisierung von 4-(tert.-Butyl)-4'-methoxydi-benzoylmethan, wobei der oder die O/W-Emulgatoren gewählt werden aus der Gruppe der Verbindungen Glycerylstearatcitrat, Glycerylstearat (selbstemulgierend), Stearinsäure, Stearatsalze, Polyglyceryl-3 methylglycose-distearat, Natriumcetearylsulfat, Kaliumcetylphosphat, Polyglyceryl-10 Stearate , Natriumstearoylglutamat und der Emulgator bei der Herstellung der Emulsion der Wasserphase zugesetzt wird.

5. Emulsion, Verfahren oder Verwendung nach einem der vorhergehenden Ansprüche, **dadurch gekennzeichnet, dass** mindestens 90 % der Öltröpfchen in der Emulsion eine Größe (Durchmesser) von über 350 nm aufweisen.

6. Emulsion, Verfahren oder Verwendung nach einem der vorhergehenden Ansprüche, **dadurch gekennzeichnet, dass** die Emulsion mit einem Rotor-/Stator-Gerät oder einem Hochdruck-Homogenisator homogenisiert wird.

7. Emulsion, Verfahren oder Verwendung nach einem der vorhergehenden Ansprüche, **dadurch gekennzeichnet, dass** die Zubereitung 4-(tert.-Butyl)-4'-methoxydiben-zoylmethan in einer Konzentration von 1,0 bis 5,0 gewichts-%, bezogen auf das Gesamtgewicht der Emulsion enthält.

8. Emulsion, Verfahren oder Verwendung nach einem der vorhergehenden Ansprüche, **dadurch gekennzeichnet, dass** die Zubereitung frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen) und 4-Methoxyzimtsäure-2-ethylhexylester (INCI Octylmethoxycinnamate).

9. Emulsion, Verfahren oder Verwendung nach einem der vorhergehenden Ansprüche, **dadurch gekennzeichnet, dass** die Zubereitung frei ist von Parabenen, Methylisothiazolinon, Chlormethylisothiazolinon und DMDM-Hydantoin

10. Emulsion, Verfahren oder Verwendung nach einem der vorhergehenden Ansprüche, **dadurch gekennzeichnet, dass** die Zubereitung einen oder mehrere UV-Filter enthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornyli-denmethyl)benzolsulfonsäuresalze; 2-Methyl-5-(2-oxo-3-bornylidenmethyl)sulfon-säuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; Ethylhexylsalicylat; Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

11. Emulsion, Verfahren oder Verwendung nach einem der vorhergehenden Ansprüche, **dadurch gekennzeichnet, dass** der oder die Emulgatoren in einer Gesamtkonzentration von 0,2 bis 5 Gewichts-%, bezogen auf das Gesamtgewicht der Zubereitung eingesetzt werden.

12. Emulsion, Verfahren oder Verwendung nach einem der vorhergehenden Ansprüche, **dadurch gekennzeichnet, dass** die Zubereitung Ethanol, Phenoxyethanol, 4-Hydroxyacetophenon, 2-Methylpropan-1,3-diol, 1,2-Pentandiol, 1,2-Hexandiol, 1,2-Octandiol und/oder 1,2-Decandiol und/oder Ethylhexylglycerin enthält.

## Claims

1. Cosmetic O/W emulsion comprising 4-(tert-butyl)-4'-methoxydibenzoylmethane, wherein at least 90% of the oil droplets in the emulsion have a size (diameter) of less than 8 µm, **characterized in that** the O/W emulsifier(s) are selected from the group of compounds comprising glyceryl stearate citrate, glyceryl stearate (self-emulsifying), stearic acid, stearate salts, polyglyceryl-3 methylglucose distearate, sodium cetearyl sulfate, potassium cetyl phosphate, polyglyceryl-10 stearate, sodium stearoyl glutamate and the emulsifier is added to the water phase in the preparation of the emulsion.

2. Process for producing cosmetic O/W emulsions comprising 4-(tert-butyl)-4'-methoxydibenzoylmethane, wherein the process comprises the following steps of:
i) combining oil phase and water phase,
ii) pre-emulsification,
iii) adding temperature-sensitive ingredients such as perfumes,
iv) post-emulsification,
wherein the post-emulsification forms oil droplets with a droplet size in which at least 90% of the oil droplets have a size (diameter) of less than 8 µm, **characterized in that** the O/W emulsifier(s) are selected from the group of compounds comprising glyceryl stearate citrate, glyceryl stearate (self-emulsifying), stearic acid, stearate salts, polyglyceryl-3 methylglucose distearate, sodium cetearyl sulfate, potassium cetyl phosphate, polyglyceryl-10 stearate, sodium stearoyl glutamate and the emulsifier is added to the water phase in the preparation of the emulsion.

3. Process for the photostabilization of 4-(tert-butyl)-4'-methoxydibenzoylmethane in cosmetic O/W emulsions, wherein the 4-(tert-butyl)-4'-methoxydibenzoylmethane is incorporated into oil droplets, which to an extent of at least 90% have a size (diameter) of less than 8 µm, **characterized in that** the O/W emulsifier(s) are selected from the group of compounds comprising glyceryl stearate citrate, glyceryl stearate (self-emulsifying), stearic acid, stearate salts, polyglyceryl-3 methylglucose distearate, sodium cetearyl sulfate, potassium cetyl phosphate, polyglyceryl-10 stearate, sodium stearoyl glutamate and the emulsifier is added to the water phase in the preparation of the emulsion.

4. Use of oil droplets with a droplet size (diameter) of less than 8 µm in cosmetic O/W emulsions for the photostabilization of 4-(tert-butyl)-4'-methoxydibenzoylmethane, wherein the O/W emulsifier(s) are selected from the group of compounds comprising glyceryl stearate citrate, glyceryl stearate (self-emulsifying), stearic acid, stearate salts, polyglyceryl-3 methylglucose distearate, sodium cetearyl sulfate, potassium cetyl phosphate, polyglyceryl-10 stearate, sodium stearoyl glutamate and the emulsifier is added to the water phase in the preparation of the emulsion.

5. Emulsion, process or use according to any of the preceding claims, **characterized in that** at least 90% of the oil droplets in the emulsion have a size (diameter) of above 350 nm.

6. Emulsion, process or use according to any of the preceding claims, **characterized in that** the emulsion is homogenized using a rotor/stator device or a high-pressure homogenizer.

7. Emulsion, process or use according to any of the preceding claims, **characterized in that** the preparation comprises 4-(tert-butyl)-4'-methoxydibenzoylmethane at a concentration of 1.0% to 5.0% by weight, based on the total weight of the emulsion.

8. Emulsion, process or use according to any of the preceding claims, **characterized in that** the preparation is free from 3-(4-methylbenzylidene)camphor, 2-hydroxy-4-methoxybenzophenone (INCI: Oxybenzone), ethylhexyl 2-cyano-3,3-diphenylacrylate (INCI: Octocrylene) and 2-ethylhexyl 4-methoxycinnamate (INCI: Octyl Methoxycinnamate).

9. Emulsion, process or use according to any of the preceding claims, **characterized in that** the preparation is free from parabens, methylisothiazolinone, chloromethylisothiazolinone and DMDM hydantoin.

10. Emulsion, process or use according to any of the preceding claims, **characterized in that** the preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; ethylhexyl salicylate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; homomenthyl salicylate; 2-ethylhexyl 2-hydroxybenzoate; dimethicodiethylbenzalmalonate; 3-(4-(2,2-bisethoxycarbonylvinyl)phenoxy)propenyl)methoxysiloxane / dimethylsiloxane copolymer; hexyl 2-(4'-diethylamino-2'-hydroxybenzoyl)benzoate; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanines; titanium dioxide; zinc oxide.

11. Emulsion, process or use according to any of the preceding claims, **characterized in that** the emulsifier(s) are used at a total concentration of 0.2% to 5% by weight, based on the total weight of the preparation.

12. Emulsion, process or use according to any of the preceding claims, **characterized in that** the preparation comprises ethanol, phenoxyethanol, 4-hydroxyacetophenone, 2-methylpropane-1,3-diol, 1,2-pentanediol, 1,2-hexanediol, 1,2-octanediol and/or 1,2-decanediol and/or ethylhexylglycerin.

## Revendications

1. Émulsion H/E cosmétique contenant du 4-(tert-butyl)-4'-méthoxydibenzoylméthane, au moins 90% des gouttes d'huile dans l'émulsion présentant une grosseur (diamètre) inférieure à 8 µm, **caractérisée en ce que** la ou les émulsifiants H/E sont choisis dans le groupe des composés stéarate-citrate de glycéryle, stéarate de glycéryle (auto-émulsifiant), acide stéarique, sels de stéarate, distéarate de polyglycéryl-3-méthylglycose, cétéarylsulfate de sodium, cétylphosphate de potassium, stéarate de polyglycéryl-10, stéarylglutamate de sodium et l'émulsifiant est ajouté à la phase aqueuse lors de la préparation de l'émulsion.

2. Procédé pour la préparation d'émulsions H/E cosmétiques contenant du 4-(tert-butyl)-4'-méthoxydibenzoylméthane, le procédé comprenant les étapes suivantes :
i) rassemblement d'une phase huileuse et d'une phase aqueuse,
ii) pré-émulsification,
iii) ajout de constituants sensibles à la température, tels que les substances parfumées,
iv) post-émulsification,
la post-émulsification formant des gouttes d'huile présentant une grosseur de goutte, au moins 90% des gouttes d'huile présentant une grosseur (diamètre) inférieure à 8 µm, **caractérisé en ce que** la ou les émulsifiants H/E sont choisis dans le groupe des composés stéarate-citrate de glycéryle, stéarate de glycéryle (auto-émulsifiant), acide stéarique, sels de stéarate, distéarate de polyglycéryl-3-méthylglycose, cétéarylsulfate de sodium, cétylphosphate de potassium, stéarate de polyglycéryl-10, stéarylglutamate de sodium et l'émulsifiant est ajouté à la phase aqueuse lors de la préparation de l'émulsion.

3. Procédé pour la photostabilisation d'émulsions H/E cosmétiques contenant du 4-(tert-butyl)-4'-méthoxydibenzoylméthane, le 4-(tert-butyl)-4'-méthoxydibenzoylméthane étant incorporé dans les gouttes d'huile qui présentent, à raison d'au moins 90%, une grosseur (diamètre) inférieure à 8 µm, **caractérisé en ce que** la ou les émulsifiants H/E sont choisis dans le groupe des composés stéarate-citrate de glycéryle, stéarate de glycéryle (auto-émulsifiant), acide stéarique, sels de stéarate, distéarate de polyglycéryl-3-méthylglycose, cétéarylsulfate de sodium, cétylphosphate de potassium, stéarate de polyglycéryl-10, stéarylglutamate de sodium et l'émulsifiant est ajouté à la phase aqueuse lors de la préparation de l'émulsion.

4. Utilisation de gouttes d'huile présentant une grosseur de goutte (diamètre) inférieure à 8 µm dans des émulsions H/E cosmétiques pour la photostabilisation du 4-(tert-butyl)-4'-méthoxydibenzoylméthane, le ou les émulsifiants H/E étant choisis dans le groupe des composés stéarate-citrate de glycéryle, stéarate de glycéryle (auto-émulsifiant), acide stéarique, sels de stéarate, distéarate de polyglycéryl-3-méthylglycose, cétéarylsulfate de sodium, cétylphosphate de potassium, stéarate de polyglycéryle-10, stéarylglutamate de sodium et l'émulsifiant étant ajouté à la phase aqueuse lors de la préparation de l'émulsion.

5. Émulsion, procédé ou utilisation selon l'une quelconque des revendications précédentes, caractérisé(e) en ce qu'au moins 90% des gouttes d'huile dans l'émulsion présentent une grosseur (diamètre) supérieure à 350 nm.

6. Émulsion, procédé ou utilisation selon l'une quelconque des revendications précédentes, caractérisé(e) en ce que l'émulsion est homogénéisée à l'aide d'un appareil à rotor-stator ou d'un homogénéisateur à haute pression.

7. Émulsion, procédé ou utilisation selon l'une quelconque des revendications précédentes, caractérisé(e) en ce que la préparation contient le 4-(tert-butyl)-4'-méthoxydibenzoylméthane en une concentration de 1,0 à 5,0% en poids, par rapport au poids total de l'émulsion.

8. Émulsion, procédé ou utilisation selon l'une quelconque des revendications précédentes, caractérisé(e) en ce que la préparation est exempte de 3-(4-méthylbenzylidène)-camphre, de 2-hydroxy-4-méthoxybenzophénone (INCI : Oxybenzon), le 2-cyano-3,3-diphénylacrylate d'éthylhexyle (INCI : Octocrylène) et d'ester 2-éthylhexylique de l'acide 4-méthoxycinnamique (INCI : Octylmethoxycinnamate).

9. Émulsion, procédé ou utilisation selon l'une quelconque des revendications précédentes, caractérisé(e) en ce que la préparation est exempte de parabènes, de méthylisothiazolinone, de chlorométhylisothiazolinone et de DMDM-hydantoïne.

10. Émulsion, procédé ou utilisation selon l'une quelconque des revendications précédentes, caractérisé(e) en ce que la préparation contient un ou plusieurs filtres UV, qui sont choisis dans le groupe de composés constitué par : l'acide 2-phénylbenzimidazole-5-sulfonique et/ou ses sels ; les sels de l'acide phénylène-1,4-bis-(2-benzimidazyl)-3,3'-5,5'-tétrasulfonique ; le 1,4-di(2-oxo-10-sulfo-3-bornylidène-méthyl)-benzène et ses sels ; les sels de l'acide 4-(2-oxo-3-bornylidène-méthyl)benzènesulfonique ; les sels de l'acide 2-méthyl-5-(2-oxo-3-bornylidène-méthyl)sulfonique ; le 2,2'-méthylène-bis-(6-(2H-benzotriazol-2-yl)-4-(1,1,3,3-tétraméthylbutyl)-phénol) ; le 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; le salicylate d'éthylhexyle ; l'acide téréphtalidène-dicamphre-sulfonique ; l'ester 2-éthylhexylique de l'acide 4-(diméthylamino)-benzoïque ; l'ester amylique de l'acide 4-(diméthylamino)benzoïque ; l'ester di(2-éthylhexylique) de l'acide 4-méthoxybenzalmalonique ; le salicylate d'homomenthyle ; le 2-hydroxybenzoate de 2-éthylhexyle ; le benzalmalonate de diméthicodiéthyle ; le copolymère de 3-(4-(2,2-bis-éthoxycarbonylvinyl)-phénoxy)propényl)-méthoxysiloxane/diméthylsiloxane ; l'ester hexylique de l'acide 2-(4'-diéthylamino-2'-hydroxybenzoyl)-benzoïque ; la dioctylbutylamidotriazone (INCI : Diethylhexyl-Butamidotriazone) ; la 2,4-bis-[5-1-(diméthylpropyl)benzoxazol-2-yl-(4-phényl)-imino]-6-(2-éthylhexyl)-imino-1,3,5-triazine de no CAS 288254-16-0) ; la 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; l'ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; la 2,4,6-tribiphényl-4-yl-1,3,5-triazine ; la mérocyanine ; le dioxyde de titane ; l'oxyde de zinc.

11. Émulsion, procédé ou utilisation selon l'une quelconque des revendications précédentes, caractérisé(e) en ce que le ou les émulsifiants sont utilisés en une concentration totale de 0,2 à 5% en poids, par rapport au poids total de la préparation.

12. Émulsion, procédé ou utilisation selon l'une quelconque des revendications précédentes, caractérisé(e) en ce que la préparation contient de l'éthanol, du phénoxyéthanol, de la 4-hydroxyacétophénone, du 2-méthylpropane-1,3-diol, du 1,2-pentanediol, du 1,2-hexanediol, du 1,2-octanediol et/ou du 1,2-décanediol et/ou de l'éthylhexylglycérol.
